# EUROPEAN PATENT SPECIFICATION

(11) **EP 2 835 355 B1**
(45) Date of publication and mention of the grant of the patent: **18.01.2017**
(21) Application number: 13768802.4
(22) Date of filing: 26.02.2013
(51) Int. Cl.: C02F 1/20, B01D 65/02, B09B 3/00, C02F 3/28, C02F 11/04, B01D 61/14, C02F 3/00, C12M 1/107, C12M 1/00, B09C 1/08, C02F 103/00, C02F 103/20, C02F 103/28, C02F 103/32

(54) **SYSTEM AND METHOD FOR TREATING ANAEROBIC TREATMENT SOLUTION**
SYSTEM UND VERFAHREN ZUR BEHANDLUNG EINER ANAEROBEN BEHANDLUNGSLÖSUNG
SYSTÈME ET PROCÉDÉ DE TRAITEMENT D'UNE SOLUTION DE TRAITEMENT ANAÉROBIE

(30) Priority: 29.03.2012 JP 2012078165
(43) Date of publication of application: 11.02.2015
(73) Proprietor: KUBOTA CORPORATION, Osaka-shi, Osaka 556-8601 (JP)
(72) Inventor: OKUMURA, Yoichi, Amagasaki-shi Hyogo 661-8567 (JP); WAKAHARA, Shin-ichiro, Amagasaki-shi Hyogo 661-8567 (JP)
(74) Representative: Müller-Boré & Partner Patentanwälte PartG mbB
(86) International application number: PCT/JP2013/054912
(87) International publication number: WO 2013/146013

(56) References cited:
- WO-A1-99/42423
- WO-A1-03/080225
- JP-A- H0 271 899
- JP-A- H0 271 899
- JP-A- H06 218 238
- JP-A- H06 218 238
- JP-A- 2003 211 194
- JP-A- 2003 211 194
- JP-A- 2004 283 658
- JP-A- 2011 230 100
- US-A1- 2005 056 590

## Description

### TECHNICAL FIELD

The present invention relates to a system for treating an anaerobically-processed liquid and a process for treating an anaerobically-processed liquid.

### BACKGROUND ART

Conventionally, a method of filtering an anaerobically-processed liquid by a submerged membrane filter is known, and filtration using the submerged membrane filter is commonly performed by directly drawing a filtrate from a filtrate discharge side of the membrane filter by a pump, as disclosed in, for example, Patent Literature 1. In addition, a biogas is generated from an anaerobically-processed liquid, and the Patent Literature 1 also discloses a generated biogas is utilized for washing a surface of the membrane filter.

Patent Literature 2 discloses a suction device which has the purpose to smoothly take out a treating water without sucking bubbles into a treating water pump by separating gas and water in the treating water in a vacuum tank and to uniformalize flux in a film separation unit by keeping the inside of a vacuum tank at a fixed negative pressure to stabilize suction efficiency.

Patent Literature 3 discloses a method of removing contaminants from water which includes the steps of first providing a water feed exposed to at least one hydrocarbon or chemical process. This water feed is set in-line with a reverse osmosis system which includes an inlet, at least one reverse osmosis membrane, a permeate outlet, and a reject outlet. Then, pressure is applied to the water feed, or where the pressure of the water feed is higher than desired, the pressure is controlled or reduced.

Patent Literature 4 discloses a waste water treating device which has the purpuse to prevent the frothing in a fermenter by providing a means for pressurizing the inside of the fermenter and a gas-liquid separator for separating and removing the generated gas dissolved in the purified water separated from treated water.

Patent Literature 5 discloses a methane fermentation method that reduces the concentration of carbon dioxide from a biogas obtained by methane fermentation without using an expensive solvent or a large amount of water, and an apparatus therefore.

### CITATION LIST

### PATENT LITERATURE

PATENT LITERATURE 1
   Japanese Unexamined Laid-open Patent Application Publication No. 2001-314839
PATENT LITERATURE 2
   Japanese Patent Application Publication No. H06 218238 A
PATENT LITERATURE 3
   United States Application Publication No. 2005/056590 A1
PATENT LITERATURE 4
   Japanese Patent Application Publication No. H02 71899 A
PATENT LITERATURE 5
   Japanese Patent Application Publication No. 2003 211194 A

### SUMMARY OF INVENTION

### TECHNICAL PROBLEM

In an anaerobic process, efficient recovery of a biogas is required; and in this respect, since the biogas is dissolved in an anaerobically-processed liquid in some degree, recovering the biogas dissolved in the anaerobically-processed liquid makes the recovery of the biogas more efficient. Further, on this occasion, it is desirable that facilities therefor are not got larger or complex.

The present invention has been achieved in view of the above circumstances, and the object of the present invention is to provide a system and process for treating an anaerobically-processed liquid, that enables efficiently conducting filtration of the anaerobically-processed liquid and recovery of a biogas, and further that makes facilities therefor compact.

### SOLUTION TO PROBLEM

This problem is solved by a system and process for treating anaerobically-processed liquid having the features of claims 1 and 4. Preferred embodiments are defined in the dependent claims, respectively.

A system for treating an anaerobically-processed liquid of the present invention which solves the above problems comprises: a solid-liquid separator provided with a filter submerged in the anaerobically-processed liquid; a gas-liquid separator retaining a filtrate passed through the filter, and having a gas-phase part which contains a gas and is positioned over the filtrate; a filtrate-flow passage connected to a filtrate discharge side of the filter and the gas-phase part of the gas-liquid separator; and a first gas-flow passage connected to the gas-phase part of the gas-liquid separator, and equipped with a depressurization means for depressurizing the gas-phase part.

According to the treatment system of the present invention, the gas-phase part of the gas-liquid separator is depressurized by the depressurization means, whereby the filtrate is deaerated in the gas-liquid separator and a biogas dissolved in the filtrate is able to be recovered. Further, since the filtrate-flow passage is connected to the filtrate discharge side of the filter and the gas-phase part of the gas-liquid separator, filtration by the filter in the solid-liquid separator is promoted by depressurizing the gas-phase part of the gas-liquid separator by the depressurization means. That is, filtration by the filter in the solid-liquid separator and deaeration of the filtrate in the gas-liquid separator are able to be performed by the single depressurization means, and therefore, the filtration of the anaerobically-processed liquid and the recovery of the biogas can be efficiently conducted, and facilities therefore can be also made compact.

Preferably, the treatment system of the present invention further comprises a second gas-flow passage connected to the first gas-flow passage on a discharge side of the depressurization means and the filtrate-flow passage on the filtrate discharge side of the filter. By providing the second gas-flow passage in this manner, the biogas discharged from the depressurization means can be fed to the filtrate discharge side of the filter through the second gas-flow passage, thereby enabling backwashing the filter. In this case, since the depressurization means acts also as a gas supply means for backwashing, backwashing of the filter can be made by providing the second gas-flow passage, resulting in saving a facility cost.

In the case of providing the second gas-flow passage, it is preferred that the treatment system of the present invention further comprises a third gas-flow passage connected to a gas-phase part of the solid-liquid separator and at least one of the first gas-flow passage on a suction side of the depressurization means and the gas-phase part of the gas-liquid separator. Furthermore, a gas holder may be provided so as to be connected to at least one of the first gas-flow passage on the suction side of the depressurization means and the gas-phase part of the gas-liquid separator, instead of or in addition to the third gas-flow passage. By providing the third gas-flow passage and/or the gas holder in these manners, a gas for backwashing can be supplied from the gas-phase part of the solid-liquid separator and/or the gas holder, thereby enabling securing a sufficient time for the backwashing.

The present invention further provides a process for treating an anaerobically-processed liquid, comprising: a filtration step of filtering the anaerobically-processed liquid containing a biogas by a submerged filter to obtain a filtrate; and a deaeration step of introducing the filtrate into a gas-liquid separator to deaerate, thereby collecting the biogas; wherein a filtrate discharge side of the filter is connected to a gas-phase part of the gas-liquid separator, and the gas-phase part of the gas-liquid separator is depressurized, whereby pressure on the filtrate discharge side of the filter is reduced to promote filtering the anaerobically-processed liquid in the filtration step and the filtrate is deaerated in the deaeration step, simultaneously.

According to the treatment process of the present invention, the gas-phase part of the gas-liquid separator is depressurized, whereby pressure on the filtrate discharge side of the filter can be reduced to promote filtering the anaerobically-processed liquid in the filtration step and the filtrate can be deaerated in the deaeration step; and hence, the filtration step and the deaeration step can be efficiently conducted.

In the deaeration step, depressurizing degree of the gas-phase part of the gas-liquid separator and/or a depth of the filtrate in the gas-liquid separator may be adjusted, thereby enabling control of a collection amount of the biogas. For example, when the depressurizing degree of the gas-phase part of the gas-liquid separator is increased (that is, pressure of the gas-phase part is reduced) or the depth of the filtrate in the gas-liquid separator is made shallow, the collection amount of the biogas can be easily increased.

It is preferred that the gas-phase part of the gas-liquid separator is depressurized by a depressurization means, and in this case, the treatment process of the present invention may further comprise a backwash step of supplying the biogas discharged from the depressurization means to the filtrate discharge side of the filter to backwash the filter. By conducting the backwash of the filter in this manner, the depressurization of the gas-phase part of the gas-liquid separator and supply of the biogas for backwashing to the filter can be performed by the single depressurization means, resulting in saving a facility cost for the backwash.

### ADVANTAGEOUS EFFECTS OF INVENTION

According to the system and process for treating an anaerobically-processed liquid of the present invention, filtration of the anaerobically-processed liquid and recovery of the biogas can be efficiently conducted and facilities therefor can be made compact.

### BRIEF DESCRIPTION OF THE DRAWINGS

Fig. 1 shows an example of a system for treating an anaerobically-processed liquid of the present invention.
Fig. 2 shows another example of a system for treating an anaerobically-processed liquid of the present invention.

### DESCRIPTION OF THE PREFERRED EMBODIMENTS

The present invention relates to a system and a process for treating an anaerobically-processed liquid, and in detail, a system and a process that enables effectively conducting filtration of an anaerobically-processed liquid and deaeration of a filtrate obtained by the filtration.

An anaerobically-processed liquid, that is a treating object in the present invention, is not particularly limited, as long as it is obtained by anaerobically-processing a liquid material (including a slurry) or a solid material containing an organic substance. Examples of the liquid or solid material containing an organic substance include, for example, sewage, night soil, livestock excreta, factory wastewater generated from food factories, paper factories or the like, kitchen waste, kitchen wastewater, plants, sludge accompanied by processing these objects, and others.

The anaerobic process may be any processes in which the liquid or solid material containing an organic substance is kept under an anaerobic condition, whereby the organic substance is fermented to generate methane. Thus, the anaerobically-processed liquid contains a biogas such as methane gas and carbon dioxide, that are methane fermentation products, and the biogas is generally dissolved in the anaerobically-processed liquid depending on its gas-phase pressure. In the present invention, the biogas may contain at least methane gas.

The anaerobically-processed liquid may be obtained after completion of methane fermentation or may be obtained in the process of methane fermentation. The methane fermentation may be conducted in an equipment designed to perform methane fermentation, such as a digestion tank (that is, a methane fermentation tank), or may be conducted by leaving the liquid or solid material containing an organic substance under an anaerobic condition during transportation or storage.

Concentration of a suspended solid (SS concentration) in the anaerobically-processed liquid is not particularly limited. For example, in the case of using sewage which has been kept under an anaerobic condition as the anaerobically-processed liquid, the SS concentration thereof is usually in the range of about 50 mg/L to 400 mg/L. In the case where the anaerobically-processed liquid derived from sewage is concentrated by a solid-liquid separator described below, the SS concentration of thus obtained anaerobically-processed liquid becomes in the range of about 1,000 mg/L to 8,000 mg/L. In the case where thickened sludge obtained by concentrating sewage or night soil is subjected to an anaerobic digestion (methane fermentation), the digested sludge obtained by the anaerobic digestion may be used as the anaerobically-processed liquid and the SS concentration of this case is in the range of about 10,000 mg/L to 50,000 mg/L. The SS concentration is measured in accordance with a wastewater standard examination method issued by the Japan Sewage Works Association.

In the present invention, first, the anaerobically-processed liquid containing a biogas is filtered by a submerged filter to obtain a filtrate (a filtration step). The filter is submerged in the anaerobically-processed liquid retained in a solid-liquid separator. The filter has a supply side and a filtrate discharge side, and the anaerobically-processed liquid is supplied to the supply side and the filtrate is drawn from the filtrate discharge side.

Filtration by the filter is performed by utilizing pressure difference between the supply side and the filtrate discharge side of the filter. In the submerged filter, filtration is promoted by water pressure on the filter; and in the present invention, pressure on the filtrate side of the filter is reduced by a depressurization means, whereby the filtration of the anaerobically-processed liquid by the filter is further promoted.

A kind of the filter is not particularly limited, and a conventionally-known filter may be used. Examples of the filter include, for example, a sand filtration filer, a membrane filter and others; and among them, a membrane filter is preferably used.

Pore size of the membrane filter is not particularly limited; however, a microfiltration membrane (MF membrane) or an ultrafiltration membrane (UF membrane), so-called, is preferably used. In respect of efficiently removing a suspended solid (SS) from the anaerobically-processed liquid, a microfiltration membrane (MF membrane) is more preferably used as the membrane filter.

A kind (shape) of the membrane filter is not particularly limited, and any membrane such as a hollow fiber membrane, a tubular membrane, a flat sheet membrane and others can be used. The membrane filter may be formed such that: the filter itself functions as a separation layer; or a deposition layer formed on a support submerged in the anaerobically-processed liquid functions as a separation layer, where the deposition layer is made by depositing a suspended solid in the anaerobically-processed liquid on the support. The latter is commonly referred to as a dynamic filtration. The separation layer is a layer defining solid-liquid separation performance and having a smallest pore size among layers constituting the membrane filter. Materials constituting the membrane filter or the support are not particularly limited; and examples of them include plastics, metals (e.g. a stainless steel), ceramics, fibrous materials, paper and others.

Any equipment is available as the solid-liquid separator for conducting filtration, if it is capable of retaining the anaerobically-processed liquid in which the filter is submerged. In the solid-liquid separator, only solid-liquid separation by the filter (that is filtration) may be conducted, or the anaerobic process may be also conducted along with the solid-liquid separation. In the former case, the solid-liquid separator may be provided so as to be connected to a digestion tank, for example. In the latter case, the filter may be installed so as to be submerged in a digestion tank or a receiving tank of sewage or night soil. In this case, the digestion tank or the receiving tank is regarded as the solid-liquid separator.

The solid-liquid separator preferably has a gas-phase part which contains a gas and is positioned over the anaerobically-processed liquid. The anaerobically-processed liquid contains a biogas, and thus, there is a possibility that the biogas may be generated from the anaerobically-processed liquid in the solid-liquid separator. Therefore, in respect of allowing the biogas generated in the solid-liquid separator to be collected, the solid-liquid separator preferably has a gas-phase part positioned over the anaerobically-processed liquid, where a gas is retained in the gas-phase part. In this case, it is preferred that the solid-liquid separator is provided with a cover and a gas outlet for recovering the biogas is provided in an upper part of the solid-liquid separator. For example, the solid-liquid separator may be constituted such that the filter placed in a tank.

The filtrate obtained by the filtration step contains a biogas dissolved therein. Therefore, in the present invention, the filtrate obtained by the filtration step is introduced into a gas-liquid separator to be deaerated, whereby the biogas is collected (a deaeration step). The gas-liquid separator retains the filtrate passed through the filter, and has a gas-phase part which contains a gas and is positioned over the filtrate. In the deaeration step, the gas-phase part of the gas-liquid separator is depressurized to deaerate the filtrate.

Any equipment is available as the gas-liquid separator, if it is capable of retaining the filtrate therein and a gas over the filtrate. The gas-liquid separator is constituted such that the gas is retained in the gas-phase part and a first gas-flow passage, which is equipped with a depressurization means, is connected to the gas-phase part of the gas-liquid separator. The gas-phase part of the gas-liquid separator is depressurized by the depressurization means. The gas-liquid separator is not restricted as long as the gas-phase part can be depressurized by the depressurization means. For example, the gas-liquid separator may comprise a tank which stores the filtrate or may comprise a pipe which the filtrate goes through. In any case, the gas-phase part needs to be provided over the filtrate in the gas-liquid separator.

Depressurization of the gas-phase part of the gas-liquid separator is performed by the depressurization means. In addition, filtration of the anaerobically-processed liquid by the filter in the solid-liquid separator is also performed by the same depressurization means, as described above. Thus, in the present invention, filtration of the anaerobically-processed liquid by the filter and deaeration of the filtrate obtained by the filtration are performed by the single depressurization means. For realizing that, a filtrate-flow passage is provided so as to be connected to the filtrate discharge side of the filter and the gas-phase part of the gas-liquid separator, in the present invention. When the filtrate-flow passage is provided in this manner, pressure on the filtrate discharge side of the filter can be reduced to promote filtering the anaerobically-processed liquid in the filtration step and the filtrate can be deaerated in the deaeration step, simultaneously, by depressurizing the gas-phase part of the gas-liquid separator. In the present invention, since the filtrate discharge side of the filter is connected to the gas-phase part of the gas-liquid separator and the filtration by the filter is performed by depressurizing the gas-phase part of the gas-liquid separator, it is possible to reduce pressure of the gas-phase part of the gas-liquid separator to almost a vacuum, thereby effectively conducting the filtration of the anaerobically-processed liquid.

The depressurization means is not particularly limited as long as it is capable of depressurizing the gas-phase part of the gas-liquid separator; however, the depressurization means preferably depressurizes the gas-phase part of the gas-liquid separator by suctioning a gas in the gas-phase part of that, and thus, a mechanical depressurization means is preferably employed. As such a depressurization means, a depressurizing pump (a vacuum pump), a blower or the like can be used.

In the filtrate-flow passage, one end of that is connected to the filtrate discharge side of the filter and the other end of that is connected to the gas-phase part of the gas-liquid separator. Arrangement of the filtrate-flow passage is not particularly limited as long as the filtrate-flow passage connects the filtrate discharge side of the filter and the gas-phase part of the gas-liquid separator. However, in view of increase in pressure difference between the supply side and the filtrate discharged side of the filter to realize the effective filtration, water level of the filtrate in the filtrate-flow passage is preferably not to be excessively high relative to water level of the anaerobically-processed liquid in the solid-liquid separator. Therefore, the water level of the filtrate in the filtrate-flow passage is preferably not located at 3 m or higher than the water level of the anaerobically-processed liquid in the solid-liquid separator, as a reference, more preferably not located at 2 m or higher than that, even more preferably 1 m or higher than that, and especially preferably located at same to or lower than the water level of the anaerobically-processed liquid in the solid-liquid separator. Thus, the filtrate-flow passage is preferably arranged so as not to extend upward from the filtrate discharge side as possible, and more preferably, the filtrate-flow passage is arranged horizontally or downslope.

In the deaeration step, a collection amount of the biogas can be controlled by adjusting depressurizing degree of the gas-phase part of the gas-liquid separator. As the depressurizing degree of the gas-phase part of the gas-liquid separator is enhanced, that is, pressure of the gas-phase part of the gas-liquid separator is reduced, the collection amount of the biogas is increased. In addition, as the depressurizing degree of the gas-phase part of the gas-liquid separator is enhanced, filtration flow rate through the filter can be increased. Therefore, the pressure of the gas-phase part of the gas-liquid separator is preferably at least lower than pressure of the gas-phase part of the solid-liquid separator; and from the aspect of conducting the filtration and the deaeration efficiently, the gas-phase part of the gas-liquid separator may be depressurized to, for example, -10 kPa (gauge pressure) or lower, preferably -30 kPa (gauge pressure) or lower, and more preferably -50 kPa (gauge pressure) or lower.

In the deaeration step, the collection amount of the biogas can be also controlled by adjusting a depth of the filtrate in the gas-liquid separator. The shallower the filtrate depth in the gas-liquid separator is, the more the deaeration can be efficiently conducted. Also, as a surface area of the filtrate contacting the gas-phase part increases, the deaeration of the filtrate can be efficiently conducted in the gas-liquid separator. Therefore, the relation between the filtrate depth in the gas-liquid separator and the surface area of the filtrate contacting the gas-phase part of the gas-liquid separator preferably satisfies the following conditions. That is, a ratio of (the surface area)^{1/2}/(the filtrate depth) as to the filtrate in the gas-liquid separator is preferably 1 or more, more preferably 2 or more, and even more preferably 4 or more. In the aforementioned ratio, the surface area means an area of the filtrate surface contacting the gas-phase part, and "(the surface area)^{1/2}" corresponds to a length of one side of a square having an area equal to that of the filtrate surface contacting the gas-phase part.

For efficiently deaerating the filtrate in the gas-liquid separator, it is also preferable to increase a contact chance between the filtrate and the gas-phase part. From this respect, the gas-liquid separator may be provided with a stirrer for stirring the filtrate or any obstacle therein, such as a filler, a projection or the like, for generating a turbulent flow in the filtrate in the gas-liquid separator. The gas-liquid separator receives the filtrate which has been filtered by the solid-liquid separator; and hence, even when the filler or the like is disposed in the gas-liquid separator, clogging is less likely to occur, resulting in reducing washing frequency to facilitate its maintenance.

By deaerating the filtrate by the depressurization means, the biogas dissolved in the filtrate moves to the gas-phase part of the gas-liquid separator, and then is discharged from the gas-liquid separator through the first gas-flow passage. The depressurization means provided in the first gas-flow passage has a suction side and a discharge side, and the biogas in the first gas-flow passage is sucked into the depressurization means from the suction side and discharged to the discharge side.

The biogas discharged to the discharge side of the depressurization means can be utilized as an energy source when it is collected. For example, the biogas may be supplied to a gas turbine to be combusted, thereby recovering energy, or methane contained in the biogas may be utilized as fuel for a fuel-cell. Of course, the biogas may be simply burned for heat utilization.

The biogas discharged to the discharge side of the depressurization means may be utilized for backwashing the filter. In this case, a second gas-flow passage is provided so as to be connected to the discharge side of the depressurization means and the filtrate discharge side of the filter, and the biogas discharged from the depressurization means is supplied to the filtrate discharge side of the filter through the second gas-flow passage, thereby enabling backwashing the filter (a backwash step). The backwashing of the filter does not need to be conducted at all times and may be conduced when the filtration flow rate through the filter is lowered. Thus, the backwashing of the filter may be conducted intermittently. When the filter is backwashed, the filtration flow rate through the filter can be allowed to be faster than that before backwashing. In addition, for enhancing the effect of the backwashing, an agent for backwashing (e.g. an oxidizer, an acid, an alkali or the like) may be fed to the filtrate discharge side of the filter along with the biogas.

The biogas discharged to the discharge side of the depressurization means may be utilized for washing a surface of the filter. In the case of using a membrane filter as the filter (but except in the case of a dynamic filtration), the biogas may be fed to under the membrane filter to conduct cross-flow washing of a surface of the membrane filter (a membrane surface).

According to the present invention, a biogas dissolved in the anaerobically-processed liquid can be efficiently recovered. Specifically, in the case of recovering the biogas from the anaerobically-processed liquid having low SS concentration, a higher percent of the biogas generated by an anaerobic process dissolves in the anaerobically-processed liquid; and therefore, according to the present invention that enables recovering the biogas dissolved in the anaerobically-processed liquid, it is possible to efficiently recover the biogas even when the anaerobically-processed liquid having SS concentration of, for example, 10,000 mg/L or lower is treated.

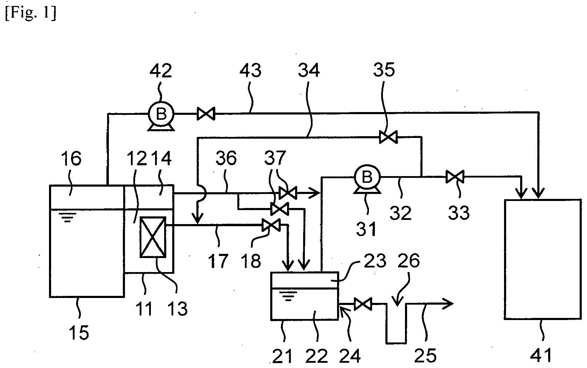

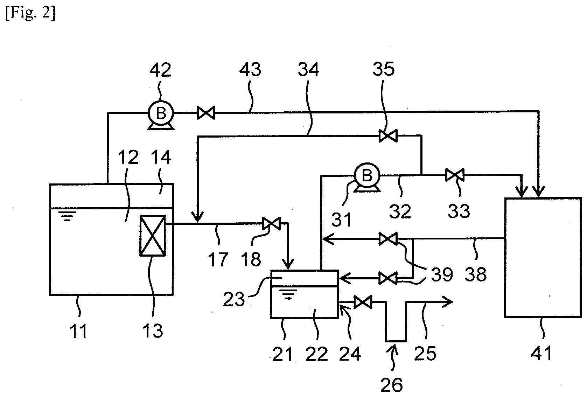

Next, examples of the process and system for treating an anaerobically-processed liquid of the present invention are explained, referring to Figures 1 and 2. However, the present invention is not limited to the embodiments shown in the Figures 1 and 2.

A system for treating an anaerobically-processed liquid shown in the Figure 1 comprises a solid-liquid separator 11 in which the anaerobically-processed liquid is filtered, a gas-liquid separator 21 in which a filtrate obtained from the solid-liquid separator 11 is deaerated, and a depressurization means 31 that depressurizes a gas-phase part 23 of the gas-liquid separator 21 to promote filtering in the solid-liquid separator 11 and deaerate in the gas-liquid separator 21, simultaneously.

The solid-liquid separator 11 is provided with a filter 13 submerged in the anaerobically-processed liquid 12 and has a gas-phase part 14 which contains a gas and is positioned over the anaerobically-processed liquid 12. In the Figure 1, a digestion tank 15 is installed upstream of and adjacent to the solid-liquid separator 11, and in the digestion tank 15, an organic wastewater is anaerobically processed to give the anaerobically-processed liquid. The anaerobically-processed liquid which has been anaerobically processed in the digestion tank 15 overflows the digestion tank 15 and flows into the solid-liquid separator 11. In the solid-liquid separator 11, the filtration step is conducted. In the filtration step, the anaerobically-processed liquid 12 in which a biogas is dissolved is filtered by a submerged filter 13 to obtain a filtrate. The digestion tank 15 may be connected to the solid-liquid separator 11 by a pipe, though it is not shown in the drawing.

The gas-phase part 14 of the solid-liquid separator 11 is connected to a gas-phase part 16 of the digestion tank 15, and a gas-flow passage 43 is provided so as to be connected to the gas-phase part 16 of the digestion tank 15. In the gas-flow passage 43, one end of that is connected to the gas-phase part 16 of the digestion tank 15 and the other end of that is connected to a gas holder 41, and the gas-flow passage 43 is equipped with a transfer means 42 (a blower in the Figure 1). In the digestion tank 15 and the solid-liquid separator 11, a biogas is generated from the organic wastewater or the anaerobically-processed liquid 12, and the thus generated biogas is transferred to the gas holder 41 by the transfer means 42 through the gas-flow passage 43 to be recovered. The one end of the gas-flow passage 43 may be connected to the gas-phase part 14 of the solid-liquid separator 11, though it is not shown in the drawing.

A filtrate-flow passage 17 is provided so as to be connected to a filtrate discharge side of the filter 13 and further connected to a gas-phase part 23 of the gas-liquid separator 21. The filtrate which has passed through the filter 13 is transferred to the gas-liquid separator 21 through the filtrate-flow passage 17.

The gas-liquid separator 21 retains the filtrate 22, and a gas is held in the gas-phase part 23 positioned over the filtrate 22. The filtrate 22 which has been introduced into the gas-liquid separator 21 is deaerated, thereby performing the deaeration step of collecting the biogas.

A first gas-flow passage 32 is connected to the gas-phase part 23 of the gas-liquid separator 21 and is equipped with a depressurization means 31 (a blower in the Figure 1). A suction side of the depressurization means 31 is connected to the gas-phase part 23 of the gas-liquid separator 21. The gas-phase part 23 of the gas-liquid separator 21 is depressurized by the depressurization means 31, whereby pressure on the filtrate discharge side of the filter 13 is reduced to promote filtering the anaerobically-processed liquid 12 in the filtration step and the filtrate 22 is deaerated in the deaeration step, simultaneously.

The biogas collected by deaerating the filtrate 22 in the gas-liquid separator 21 is carried to a discharge side of the depressurization means 31 through the first gas-flow passage 32. The gas holder 41 is connected to the first gas-flow passage 32 on the discharge side of the depressurization means 31, and the biogas discharged from the discharge side of the depressurization means 31 is transferred to the gas holder 41 to be recovered.

In the treatment system shown in the Figure 1, the biogas generated from the digestion tank 15 or the solid-liquid separator 11 is separately recovered from that from the gas-liquid separator 21. Comparing compositions of these biogases, the biogas recovered from the gas-liquid separator 21 tends to contains a higher percentage of carbon dioxide than that from the digestion tank 15 or the solid-liquid separator 11, since solubility of carbon dioxide in the filtrate 22 is higher than that of methane. Therefore, for example, for increasing a percentage of methane gas in the biogas collected in the gas holder 41, depressurizing degree of the gas-phase part 23 of the gas-liquid separator 21 may be decreased (that is, pressure of the gas-phase part 23 may be raised); and for increasing an amount of recovering the methane gas from the anaerobically-processed liquid 12 (including the filtrate 22), the depressurizing degree of the gas-phase part 23 of the gas-liquid separator 21 may be increased (that is, pressure of the gas-phase part 23 may be reduced). On actual operation of the treatment system, it is preferred that the depressurizing degree of the gas-phase part 23 of the gas-liquid separator 21 is appropriately adjusted in consideration of both the proportion of methane gas in the biogas and the recovered amount of methane gas. The composition and amount of the biogas to be collected in the gas holder 41 can be also controlled by adjusting a depth of the filtrate appropriately in the same manner as adjusting the depressurizing degree.

The filtrate 22 which was deaerated in the gas-liquid separator 21 is discharged through a filtrate outlet port 24. It is preferred that a filtrate-outlet passage 25 is connected to the filtrate outlet port 24 and sealed by water at a water sealing part 26 so that airtightness in the gas-liquid separator 21 is kept. The filtrate discharged through the filtrate-outlet passage 25 may be released as is or may be subjected to another treatment.

In the Figure 1, a second gas-flow passage 34 is provided so as to be connected to the first gas-flow passage 32 on the discharge side of the depressurization means 31 and the filtrate-flow passage 17 on the filtrate discharge side of the filter 13. When a valve 33 provided in the first gas-flow passage 32 is closed, a valve 35 provided in the second gas-flow passage 34 is opened, and a valve 18 provided in the filtrate-flow passage 17 is closed, the biogas discharged from the depressurization means 31 can be fed to the filtrate discharge side of the filter 13 through the second gas-flow passage 34, thereby enabling backwashing the filter 13. In this case, since the depressurization means 31 also acts as a gas supply means for backwashing, backwashing of the filter 13 is realized only by providing the second gas-flow passage 34 for backwashing, resulting in saving a facility cost.

In the case where the second gas-flow passage 34 for backwashing is provided, it is preferred that a third gas-flow passage 36 is provided so as to be connected to the gas-phase part 14 of the solid-liquid separator 11 and at least one of the first gas-flow passage 32 on the suction side of the depressurization means 31 and the gas-phase part 23 of the gas-liquid separator 21. When the third gas-flow passage 36 is provided in this manner, a gas for backwashing can be supplied from the gas-phase part 14 of the solid-liquid separator 11 by opening a valve 37 provided in the third gas-flow passage 36, thereby enabling securing a sufficient time for the backwashing.

Figure 2 shows another treatment system that is different from the system for treating an anaerobically-processed liquid shown in the Figure 1. In the below explanation on the treatment system shown in the Figure 2, explanations overlapping the above explanations regarding the Figure 1 are omitted.

In the system for treating an anaerobically-processed liquid shown in the Figure 2, the solid-liquid separator 11 doubles as a digestion tank for performing anaerobic digestion. Thus, an organic wastewater is supplied to the solid-liquid separator 11 and anaerobically processed therein to give the anaerobically-processed liquid 12. For example, in the case where the anaerobic digestion is conducted in a relatively small scale to obtain the anaerobically-processed liquid, the solid-liquid separator 11 may double as the digestion tank, thereby enabling saving a facility cost.

In the Figure 2, a fourth gas-flow passage 38 is provided so as to be connected to the gas holder 41 and at least one of the first gas-flow passage 32 on the suction side of the depressurization means 31 and the gas-phase part 23 of the gas-liquid separator 21, instead of the third gas-flow passage 36. Thus, the gas holder 41 is connected to at least one of the first gas-flow passage 32 on the suction side of the depressurization means 31 and the gas-phase part 23 of the gas-liquid separator 21. When the fourth gas-flow passage 38 is provided in this manner, a gas for backwashing is supplied from the gas holder 41 by opening a valve 39 provided in the fourth gas-flow passage 38, thereby enabling securing a sufficient time for the backwashing.

### INDUSTRIAL APPLICABILITY

The present invention is available for treating sewage, night soil, livestock excreta, factory wastewater generated from food factories, paper factories or the like, kitchen waste, kitchen wastewater, plants, sludge accompanied by processing these objects, or others.

### REFERENCE SIGNS LIST

- 11:: a solid-liquid separator
- 12:: an anaerobically-processed liquid
- 13:: a filter
- 14:: a gas-phase part
- 17:: a filtrate-flow passage
- 21:: a gas-liquid separator
- 22:: a filtrate
- 23:: a gas-phase part
- 31:: a depressurization means
- 32:: a first gas-flow passage
- 34:: a second gas-flow passage
- 36:: a third gas-flow passage
- 41:: a gas holder

## Claims

1. A system for treating an anaerobically-processed liquid (12), comprising:
a solid-liquid separator (11) provided with a filter (13) submerged in the anaerobically-processed liquid (12);
a gas-liquid separator (21) retaining a filtrate (22) passed through the filter (13), and having a gas-phase part (23) which contains a gas and is positioned over the filtrate (22);
a filtrate-flow passage (17) connected to a filtrate discharge side of the filter (13) and the gas-phase part (23) of the gas-liquid separator (21);
a first gas-flow passage (32) connected to the gas-phase part (23) of the gas-liquid separator (21), and equipped with a depressurization means (31) for depressurizing the gas-phase part (23); and
**characterised by**
a second gas-flow passage (34) connected to the first gas-flow passage (32) on a discharge side of the depressurization means (31) and the filtrate-flow passage (17) on the filtrate discharge side of the filter (13).

2. The system according to claim 1, wherein
the solid-liquid separator (11) has a gas-phase part (14) which contains a gas and is positioned over the anaerobically-processed liquid (12), and
a third gas-flow passage (36) is connected to the gas-phase part (14) of the solid-liquid separator (11) and at least one of the first gas-flow passage (32) on a suction side of the depressurization means (31) and the gas-phase part (23) of the gas-liquid separator (21).

3. The system according to claim 1 or 2, further comprising
a gas holder (41) connected to at least one of the first gas-flow passage (32) on the suction side of the depressurization means (31) and the gas-phase part (23) of the gas-liquid separator (21).

4. A process for treating an anaerobically-processed liquid (12), comprising:
a filtration step of filtering the anaerobically-processed liquid (12) containing a biogas by a submerged filter (13) to obtain a filtrate (22); and
a deaeration step of introducing the filtrate (22) into a gas-liquid separator (21) to deaerate, thereby collecting the biogas;
wherein a filtrate discharge side of the filter (13) is connected to a gas-phase part (23) of the gas-liquid separator (21), and
the gas-phase part (23) of the gas-liquid separator (21) is depressurized by a depressurization means (31), whereby pressure on the filtrate discharge side of the filter (13) is reduced to promote filtering the anaerobically-processed liquid (12) in the filtration step and the filtrate (22) is deaerated in the deaeration step, simultaneously, and
**characterised in that**
the process further comprises a backwash step of supplying the biogas discharged from the depressurization means (31) to the filtrate discharge side of the filter (13) to backwash the filter (13).

5. The process according to claim 4, wherein
depressurizing degree of the gas-phase part (23) of the gas-liquid separator (21) and/or a depth of the filtrate (22) in the gas-liquid separator (21) is adjusted in the deaeration step to control a collection amount of the biogas.

## Patentansprüche

1. System zum Behandeln einer anaerob verarbeiteten Flüssigkeit (12), umfassend:
einen Feststoff-Flüssigkeit-Separator (11), der mit einem Filter (13) versehen ist, der in die anaerob verarbeitete Flüssigkeit getaucht ist;
einen Gas-Flüssigkeit-Separator (21), der ein Filtrat (22) zurückhält, das durch den Filter (13) geleitet wird, und der einen Gasphasenteil (23) aufweist, der ein Gas enthält und über dem Filtrat (22) positioniert ist;
einen Filtratstromdurchgang (17), der mit einer Filtratauslassseite des Filters (13) und dem Gasphasenteil (23) des Gas-Flüssigkeit-Separators verbunden ist;
einen ersten Gasstromdurchgang (32), der mit dem Gasphasenteil (23) des Gas-Flüssigkeit-Separators (21) verbunden ist und mit einem Druckverminderungsmittel (31) zum Vermindern des Drucks des Gasphasenteils (23) ausgestattet ist; und
**gekennzeichnet durch**
einen zweiten Gasstromdurchgang (34), der mit dem ersten Gasstromdurchgang (32) auf einer Auslassseite des Druckverminderungsmittels (31) und dem Filtratstromdurchgang (17) auf der Filtratauslassseite des Filters (13) verbunden ist.

2. System nach Anspruch 1, wobei
der Feststoff-Flüssigkeit-Separator (11) einen Gasphasenteil (14) aufweist, der ein Gas enthält und über der anaerob verarbeiteten Flüssigkeit (12) positioniert ist, und
ein dritter Gasstromdurchgang (36) mit dem Gasphasenteil (14) des Feststoff-Flüssigkeit-Separators (11) und zumindest einem des ersten Gasstromdurchgangs (32) auf einer Saugseite des Druckverminderungsmittels (31) und des Gasphasenteils (23) des Gas-Flüssigkeit-Separators (21) verbunden ist.

3. System nach Anspruch 1 öder 2, ferner umfassend
einen Gashalter bzw. Gasbehälter (41), der mit zumindest einem des ersten Gasstromdurchgangs (32) auf der Saugseite des Druckverminderungsmittels (31) und des Gasphasenteils (23) des Gas-Flüssigkeit-Separators (21) verbunden ist.

4. Verfahren zum Behandeln einer anaerob verarbeiteten Flüssigkeit (12), umfassend:
einen Filtrationsschritt des Filterns der anaerob verarbeiteten Flüssigkeit (12), die ein Biogas enthält, durch einen eingetauchten Filter (13), um ein Filtrat (22) zu erhalten; und
einen Entlüftungsschritt des Einbringens des Filtrats (22) in einen Gas-Flüssigkeit-Separator (21) zum Entlüften, wodurch das Biogas gesammelt wird;
wobei eine Filtratauslassseite des Filters (13) mit einem Gasphasenteil (23) des Gas-Flüssigkeit-Separators (21) verbunden ist, und
der Gasphasenteil (23) des Gas-Flüssigkeit-Separators (21) durch ein Druckverminderungsmittel (31) im Druck vermindert wird, wodurch Druck auf der Filtratauslassseite des Filters (13) reduziert wird, um das Filtrieren der anaerob verarbeiteten Flüssigkeit (12) in dem Filtrationsschritt zu fördern, und das Filtrat (22) in dem Entlüftungsschritt gleichzeitig entlüftet wird, und
**dadurch gekennzeichnet, dass**
das Verfahren ferner einen Rückspülschritt des Zuführens des von dem Druckverminderungsmittel (31) ausgestoßenen Biogases an die Filtratauslassseite des Filters (13) umfasst, um den Filter (13) rückzuspülen.

5. Verfahren nach Anspruch 4, wobei ein Druckverminderungsgrad des Gasphasenteils (23) des Gas-Flüssigkeit-Separators (21) und/oder eine Tiefe des Filtrats (22) in dem Gas-Flüssigkeit-Separator (21) in dem Entlüftungsschritt eingestellt wird bzw. werden, um eine Sammelmenge des Biogases zu steuern.

## Revendications

1. Système de traitement d'un liquide transformé par voie anaérobie (12) comprenant :
un séparateur solide-liquide (11) pourvu d'un filtre (13) submergé dans le liquide transformé par voie anaérobie (12) ;
un séparateur gaz-liquide (21) retenant un filtrat (22) passé à travers le filtre (13) et ayant une partie à phase gazeuse (23) qui contient un gaz et est positionnée sur le filtrat (22) ;
un passage d'écoulement de filtrat (17) connecté à un côté d'évacuation de filtrat du filtre (13) et à la partie à phase gazeuse (23) du séparateur gaz-liquide (21) ;
un premier passage d'écoulement gazeux (32) connecté à la partie à phase gazeuse (23) du séparateur gaz-liquide (21) et équipé d'un moyen de dépressurisation (31) pour dépressuriser la partie à phase gazeuse (23) ; et
**caractérisé par**
un deuxième passage d'écoulement gazeux (34) connecté au premier passage d'écoulement gazeux (32) sur un côté d'évacuation du moyen de dépressurisation (31) et au passage d'écoulement de filtrat (17) sur le côté d'évacuation de filtrat du filtre (13).

2. Système selon la revendication 1, dans lequel
le séparateur solide-liquide (11) a une partie à phase gazeuse (14) qui contient un gaz et est positionnée sur le liquide transformé par voie anaérobie (12), et
un troisième passage d'écoulement gazeux (36) est connecté à la partie à phase gazeuse (14) du séparateur solide-liquide (11) et à au moins un du premier passage d'écoulement gazeux (32) sur un côté d'aspiration du moyen de dépressurisation (31) et de la partie à phase gazeuse (23) du séparateur gaz-liquide (21).

3. Système selon la revendication 1 ou 2, comprenant en outre un gazomètre (41) connecté à au moins un du premier passage d'écoulement gazeux (32) sur le côté d'aspiration du moyen de dépressurisation (31) et de la partie à phase gazeuse (23) du séparateur gaz-liquide (21).

4. Procédé de traitement d'un liquide transformé par voie anaérobie (12) comprenant :
une étape de filtration consistant à filtrer le liquide transformé par voie anaérobie (12) contenant un biogaz par un filtre submergé (13) pour obtenir un filtrat (22) ; et
une étape de désaération consistant à introduire le filtrat (22) dans un séparateur gaz-liquide (21) pour désaérer, recueillant de ce fait le biogaz ;
dans lequel un côté d'évacuation de filtrat du filtre (13) est connecté à une partie à phase gazeuse (23) du séparateur gaz-liquide (21), et
la partie à phase gazeuse (23) du séparateur gaz-liquide (21) est dépressurisée par un moyen de dépressurisation (31), moyennant lequel la pression sur le côté d'évacuation de filtrat du filtre (13) est réduite pour promouvoir la filtration du liquide transformé par voie anaérobie (12) dans l'étape de filtration et le filtrat (22) est désaéré dans l'étape de désaération, simultanément, et
**caractérisé en ce que**
le procédé comprend en outre une étape de lavage à contre-courant consistant à fournir le biogaz évacué du moyen de dépressurisation (31) au côté d'évacuation de filtrat du filtre (13) pour laver le filtre (13) à contre-courant.

5. Procédé selon la revendication 4, dans lequel
le degré de dépressurisation de la partie à phase gazeuse (23) du séparateur gaz-liquide (21) et/ou une profondeur du filtrat (22) dans le séparateur gaz-liquide (21) est ajusté(e) dans l'étape de désaération pour commander une quantité de recueil du biogaz.
